# EUROPÄISCHE PATENTSCHRIFT

(11) **EP 3 355 987 B1**
(45) Veröffentlichungstag und Bekanntmachung des Hinweises auf die Patenterteilung: **27.02.2019**
(21) Anmeldenummer: 16774507.4
(22) Anmeldetag: 29.09.2016
(51) Int. Cl.: A61N 2/00, A61N 2/02

(54) **MAGNETISCHE STIMULATIONSVORRICHTUNG**
MAGNETIC STIMULATION DEVICE
DISPOSITIF DE STIMULATION MAGNÉTIQUE

(30) Priorität: 02.10.2015 AT 508382015
(43) Veröffentlichungstag der Anmeldung: 08.08.2018
(73) Patentinhaber: Pontemed AG, 9053 Teufen (CH)
(72) Erfinder: MAYR, Winfried, 2340 Mödling (AT)
(74) Vertreter: Sonn & Partner Patentanwälte
(86) Internationale Anmeldenummer: PCT/EP2016/073292
(87) Internationale Veröffentlichungsnummer: WO 2017/055471

(56) Entgegenhaltungen:
- EP-A2- 0 850 665
- DE-A1-102008 034 237
- US-A1- 2004 254 492
- US-A1- 2009 216 067
- US-A1- 2010 256 439
- US-B1- 6 210 317

## Beschreibung

Die Erfindung betrifft eine Vorrichtung zur magnetischen Stimulation von Regionen eines menschlichen oder tierischen Körpers nach dem Oberbegriff des Anspruchs 1.

Im Gegensatz zur funktionellen Elektrostimulation (FES), bei der ein Muskel oder Nerv zur Durchführung einer Muskelkontraktion oder zur Beeinflussung anderer Nervenfunktionen über kontaktierende Elektroden elektrisch stimuliert wird, um bestimmte physiologische Vorgänge zu unterstützen bzw. zu ersetzen, wird bei der funktionellen Magnetstimulation (FMS) eine Nervenaktivierung, die beispielsweise zu einer Muskelkontraktion führen kann, durch entsprechende Magnetfelder berührungslos ausgelöst.

Die funktionelle Magnetstimulation hat gegenüber funktioneller Elektrostimulation mit an der Hautoberfläche liegenden Elektroden den wesentlichen Vorteil, dass in der Haut liegende Schmerzsensoren wesentlich geringer aktiviert werden und die Anwendung bei vergleichbarer neuromuskulärer Aktivierung als wesentlich angenehmer empfunden wird. Dies beruht auf der Tatsache, dass die Schmerzsensoren in im Vergleich zu tieferliegenden Gewebeanteilen höherohmigen Gewebeschichten liegen. Der Stromfluss bei elektrischer Stimulation bewirkt daher relativ hohe elektrische Feldstärken besonders im Bereich der Schmerzsensorik, während die wirkungsrelevanten induzierten Wirbelströme bei magnetischer Stimulation im niederohmigen tieferliegenden Gewebe wesentlich stärker ausgeprägt sind als in oberflächennahen höherohmigen Gewebe.

Weiters ist bei der funktionellen Magnetstimulation der Aufwand und das Risiko durch den Wegfall der häufig notwendigen Implantation von Nerven- oder Muskelelektroden bei der funktionellen Elektrostimulation wesentlich niedriger und die Akzeptanz höher. Demgegenüber ist jedoch die gezielte Stimulation bestimmter Nerven oder Muskeln über das Magnetfeld schwieriger als bei der direkten elektrischen Stimulation mit Hilfe von Hautelektroden oder implantierten Elektroden. Insbesondere ist es bei der Stimulation tieferliegender Regionen sehr schwierig bestimmte Punkte, sogenannte motorische Reizpunkte oder Motorpoints mit dem Magnetfeld zu erreichen und beispielsweise die Kontraktion der gewünschten Muskeln zu erzielen.

Die EP 0 850 665 A2 beschreibt eine magnetische Stimulationsvorrichtung zur Inkontinenzbehandlung, wobei eine Verstellvorrichtung zur Anpassung der Vorrichtung an den Patienten vorgesehen ist.

Die US 2010/0256439 A1 beschreibt eine Vorrichtung und ein Verfahren zum Triggern von Elektromagneten, welche für die transkranielle magnetische Stimulation verwendet werden. Zur Anpassung der Vorrichtung an die individuelle Kopfform des Patienten ist eine Verstellung der Magnetspulen möglich.

Die US 2009/0216067 A1 betrifft einen Roboter zur Positionierung und Bewegung einer Behandlungsvorrichtung in Bezug auf einen zu behandelnden Körper, wobei die Ausrichtung eines Instruments in Bezug auf die Position und Größe des jeweiligen Patienten Gegenstand der Erfindung ist.

Ein Beispiel für eine Vorrichtung zur magnetischen Stimulation wird in der WO 2009/126117 A1 beschrieben. Dabei wird mit Hilfe einer Magnetspule ein Magnetfeld in tiefere Gewebeschichten induziert, wodurch eine Depolarisation neuronaler Zellen resultiert, welche zu Muskelkontraktionen bestimmter Muskeln in bestimmten Körperregionen führen.

Ein weiteres Verfahren und eine Vorrichtung zur neuromagnetischen Stimulation ist aus der EP 0 617 982 A1 bekannt geworden, wobei dem Magnetfeld ein fokussierter Ultraschallstrahl überlagert wird, wodurch eine genauere räumliche Stimulation ermöglicht werden soll.

Ein Verfahren und eine Vorrichtung für das Beckenbodentraining mit Hilfe magnetischer Stimulation ist beispielsweise aus der DE 10 2012 012 149 A1 bekannt geworden. Dabei wird zusätzlich zur Magnetstimulation dem Gewebe noch Sauerstoff und bzw. oder Ozon zugeführt um das Training und den Aufbau der Muskulatur noch weiter zu unterstützen.

Weitere Vorrichtungen zur magnetischen Stimulation von Regionen eines menschlichen oder tierischen Körpers sind beispielsweise aus der US 2012/0197063 A1, der US 2012/0289764 A1, der WO 00/02619 A2, der DE 102 42 542 A1 oder der US 4,428,366 A bekannt geworden. Dabei werden die zur Erzeugung des Magnetfelds notwendigen Magnetspulen beiderseits der zu stimulierenden Körperregion angeordnet.

Auch die JP 2002-233575 A zeigt eine Vorrichtung zur magnetischen Stimulation für medizinische Anwendungen, wobei die Magnetspulen entsprechend der zu stimulierenden Körperregion anordenbar sind.

Die Aufgabe der vorliegenden Erfindung besteht darin, eine oben genannte Vorrichtung zur magnetischen Stimulation zu Schaffen, durch welche eine optimale Richtung des magnetischen Felds auf die jeweiligen motorischen Reizpunkte und somit eine optimale Stimulation erzielt werden kann. Gleichzeitig soll die Erfindung die Magnetfeldeinwirkung derart konzentrieren, dass es möglichst selektiv auf das zu stimulierenden Gewebeareal einwirkt, jedoch benachbarte Areale, in dem z.B. Implantate liegen, weitgehend ausspart. Nachteile bekannter Stimulationsvorrichtungen sollen verhindert oder zumindest reduziert werden.

Gelöst wird die erfindungsgemäße Aufgabe dadurch, dass die zumindest zwei als Luftspulen ausgebildeten Magnetspulen oder die Pole des Magnetjochs über die Verstellvorrichtung verstellbar miteinander verbunden sind, sodass eine Anpassung an die Größe der zu stimulierenden Körperregion erzielbar ist, und dass zumindest ein Antrieb zur Verstellung des Drehgelenks und bzw. oder des Schiebegelenks vorgesehen ist, welcher zumindest eine Antrieb mit zumindest einem Sensor zur Erfassung eines Drucks auf die zu stimulierende Körperregion und einer Bewegung der zu stimulierenden Körperregion verbunden ist, für eine automatische, aktive Nachführung der zumindest einen Magnetspule oder der Pole des Magnetjochs synchron an die zu stimulierende bewegte Körperregion. Gemäß einer ersten Ausführungsvariante der Stimulationsvorrichtung sind zumindest zwei als Luftspulen ausgebildete Magnetspulen vorgesehen, welche beiderseits der zu stimulierenden Körperregion anordenbar und über die Verstellvorrichtung verstellbar miteinander verbunden sind. Die zumindest zwei als Luftspulen ausgebildeten Magnetspulen schließen somit das zu stimulierende Areal ein und bewirken eine entsprechende Konzentration des Magnetfelds in den tieferliegenden Körperregionen, wo die zu stimulierenden motorischen Reizpunkte oder Motorpoints angeordnet sind. Durch die verstellbare Anordnung der Luftspulen kann die zu simulierende Körperregion, beispielsweise eine Extremität, optimal zwischen den Magnetspulen angeordnet und ein hochkonzentriertes Magnetfeld in dieser Körperregion und somit eine optimale Stimulation erzielt werden. Luftspulen zeichnen sich durch ein niedriges Gewicht aber auch ein größeres Streufeld aus. Gemäß einer zweiten Ausführungsvariante ist die zumindest eine Magnetspule auf einem Magnetjoch angeordnet, wobei die Pole des Magnetjochs beiderseits der zu stimulierenden Körperregion anordenbar und über die Verstellvorrichtung verstellbar miteinander verbunden sind. Im Falle dieser Ausführungsvariante wird die zu stimulierende Körperregion zwischen den Polen des Magnetjochs angeordnet und eine Anpassung an die Größe der zu stimulierenden Körperregion durch entsprechende Verstellung der Pole des Magnetjochs erzielt. Auch in diesem Fall kann eine gezielte Konzentration des stimulierenden Magnetfelds in den tieferliegenden Körperregionen und somit eine optimale Stimulation der jeweiligen Motorpoints erreicht werden. Die Ausführung mit Magnetjoch weist ein kleineres Streufeld aber auch ein höheres Gewicht als Luftspulen auf. Durch die verstellbare Anordnung der zumindest einen Magnetspule gegenüber der zu stimulierenden Körperregion kann eine optimale Anordnung der zumindest einen Magnetspule in Bezug auf die zu stimulierende Körperregion erzielt und somit eine optimale Stimulation der jeweiligen motorischen Reizpunkte oder Motorpoints bewirkt werden. Die Verstellvorrichtung wird entsprechend an die zu stimulierende Körperregion angepasst. Für kompliziertere Adaptierungen an die Form der jeweiligen Körperregion ist die Kombination eines Drehgelenks mit einem Schiebegelenk vorgesehen Auf diese Weise kann beispielsweise eine atmungssynchrone Bewegung der Stimulationsvorrichtung bei einer Stimulation im Bereich des Brustkorbes bzw. des Zwerchfells erfolgen. Gemäß der Erfindung ist zumindest ein Antrieb zur Verstellung des Drehgelenks und bzw. oder des Schiebegelenks vorgesehen. Durch einen derartigen Antrieb kann eine automatische Anpassung der Position der Magnetspulen bzw. Pole des Magnetjochs erfolgen. Dadurch könnte beispielsweise im Falle einer Stimulation im Bereich des Brustkorbes bzw. des Zwerchfells eine motorische Bewegung synchron zur Atmung durchgeführt werden. Als Antriebe kommen insbesondere geeignete Elektromotoren zur Anwendung.

Der zumindest eine Antrieb ist mit zumindest einem Sensor verbunden, um entsprechende Regelungen der Antriebe zu ermöglichen.

Zumindest ein Sensor ist durch einen Drucksensor gebildet. Dadurch kann ein unzulässig hoher Druck auf die zu stimulierende Körperregion vermieden werden oder das Anliegen der Magnetspule oder des Magnetpols an der Körperoberfläche erkannt werden.

Die Verstellvorrichtung kann im Falle der beiderseits zu stimulierenden Körperregion angeordneten zumindest zwei Luftspulen aus magnetisch nicht leitfähigem bzw. magnetisch isolierendem Material, insbesondere Kunststoff, gebildet sein. Dadurch wird ein magnetischer Kurzschluss der von den zumindest zwei Luftspulen ausgehenden magnetischen Feldlinien vermieden. Darüber hinaus ist Kunststoff billig herstellbar und weist ein niedriges Gewicht auf.

Im Falle der Verwendung zumindest einer auf einem Magnetjoch angeordneten Magnetspule zur Erzeugung des magnetischen Feldes ist die Verstellvorrichtung aus magnetisch leitfähigem Material, beispielsweise Eisen, gebildet, sodass sie einen Teil des magnetischen Kreises bzw. Magnetjochs bilden kann.

Weiteres kann zumindest ein Sensor durch einen optischen Sensor gebildet sein der beispielsweise die Annäherung an die Körperoberfläche der zu stimulierenden Körperregion erkennt oder auch Bewegungen des Patienten erfasst.

Schließlich kann zumindest ein Sensor durch einen Atemflowsensor gebildet sein, sodass eine Bewegung der Verstellvorrichtung synchron zur Spontanatmung des Patienten vorgenommen werden kann. Bei Patienten mit Spontanatmung kann über die Magnetstimulation eine Unterstützung der Atmung erzielt werden.

Um die Behandlung für den Patienten bequemer gestalten zu können, kann eine Liege oder ein Sitz vorgesehen sein, wobei unterhalb der Liege oder des Sitzes allenfalls in einer entsprechenden Anpassung zumindest eine Magnetspule angeordnet ist. Auf diese Weise können auch liegende oder sogar schlafende Patienten oder Intensivpatienten stimuliert werden. Beispielsweise kann eine Stimulation des Zwerchfells während der Intensivpflege vorgenommen werden, z.B. um den Patienten auf die Entwöhnung vom Respirator vorzubereiten. Es wäre auch eine nichtinvasive Beatmung ohne Intubation oder Tracheostoma denkbar, zum Beispiel temporär in der Notfallmedizin oder postoperativen Beatmung oder auch über längere Zeiträume bei Intensivpatienten.

Eine noch gezieltere Stimulation kann dadurch erreicht werden, dass Elemente zum Konzentrieren des Magnetfelds vorgesehen sind. Dabei kommen verschiedene Elemente zur Konzentration des Magnetfelds, insbesondere Konzentrationsspulen oder Permanentmagnete, zur Anwendung.

Die Erfindung wird anhand der beigefügten Zeichnungen, welche Ausführungsbeispiele der Erfindung zeigen, näher erläutert. Darin zeigen:
- Fig. 1: eine Prinzipskizze der funktionellen magnetischen Stimulation tieferer Körperregionen;
- Fig. 2: ein Blockschaltbild einer Ausführungsvariante der erfindungsgemäßen magnetischen Stimulationsvorrichtung mit zwei als Luftspulen ausgeführten Magnetspulen;
- Fig. 3: ein Blockschaltbild einer weiteren Ausführungsvariante der erfindungsgemäßen magnetischen Stimulationsvorrichtung mit einer Magnetspule und einem Magnetjoch;
- Fig. 4: eine Prinzipskizze einer Liege mit einer erfindungsgemäßen magnetischen Stimulationsvorrichtung; und
- Fig. 5: eine Detailansicht einer magnetischen Stimulationsvorrichtung mit Elementen zur Konzentration des Magnetfelds.

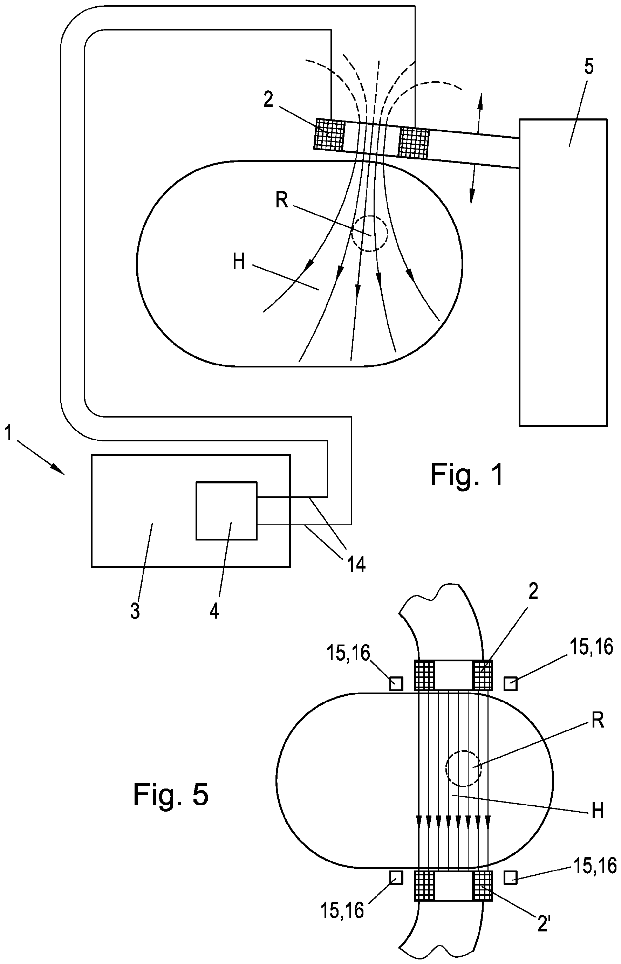

Fig. 1 zeigt eine Prinzipskizze der funktionellen magnetischen Stimulation tieferer Körperregionen. Die Vorrichtung 1 zur magnetischen Stimulation von Regionen R eines menschlichen oder tierischen Körpers beinhaltet zumindest eine Magnetspule 2, welche über entsprechende Anschlussleitungen 14 mit einem Stimulator 3 verbunden ist, der einen Leistungsteil 4 aufweist. Der Leistungsteil 4 erzeugt die elektrischen Impulse oder Signale, welche an die zumindest eine Magnetspule 2 angelegt werden. Derartige Impulse weisen idealerweise Rechteckform auf, können aber für bestimmte Anwendungen auch andere Formen, wie z.B. Dreiecksform oder Sinusform, besitzen, durch welche Wechselfelder in der Magnetspule 2 erzeugt werden können. Durch die elektrischen Impulse wird in der zumindest einen Magnetspule 2 ein Magnetfeld H erzeugt, welches in der Körperregion R induziert wird und dort an gewünschten Punkten, sog. Motorpoints, Wirkungen hervorruft, die beispielsweise zu Muskelkontraktionen der gewünschten Körperregion R führen können. Beispielsweise kann durch eine derartige funktionelle magnetische Stimulation des Zwerchfells eine Beatmung eines Patienten durchgeführt werden. Auf diese Weise kann die Respiratorabhängigkeit eines Patienten hinausgezögert oder sogar vermieden werden. Insbesondere für die postoperative Beatmung eines Patienten stellt diese nicht-invasive Methode eine optimale Alternative zum Respirator dar. Aufgrund einer sehr raschen Degeneration des Zwerchfells ist die Entwöhnung vom Respirator bereits nach wenigen Tagen sehr aufwendig, weshalb eine Vermeidung der Respiratorabhängigkeit anzustreben ist.

Um eine konzentriertere Anordnung des Magnetfelds H in der zu stimulierenden Region R zu erzielen, ist die zumindest eine Magnetspule 2 erfindungsgemäß mit einer Verstellvorrichtung 5 verbunden, um die zumindest eine Magnetspule 2 gegenüber der zu stimulierenden Körperregion R verstellen zu können. Somit kann eine optimale Anpassung des Magnetfelds H in Bezug auf die zu stimulierende Region R vorgenommen werden.

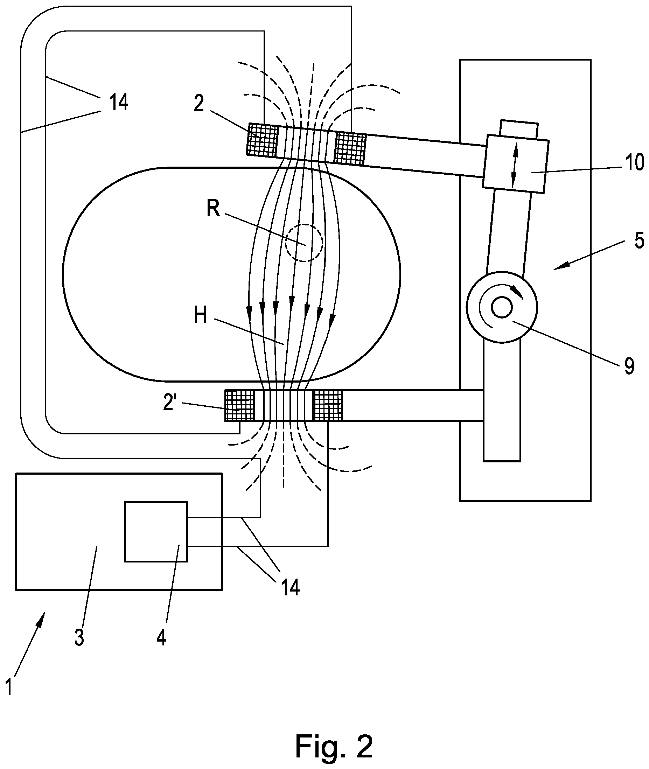

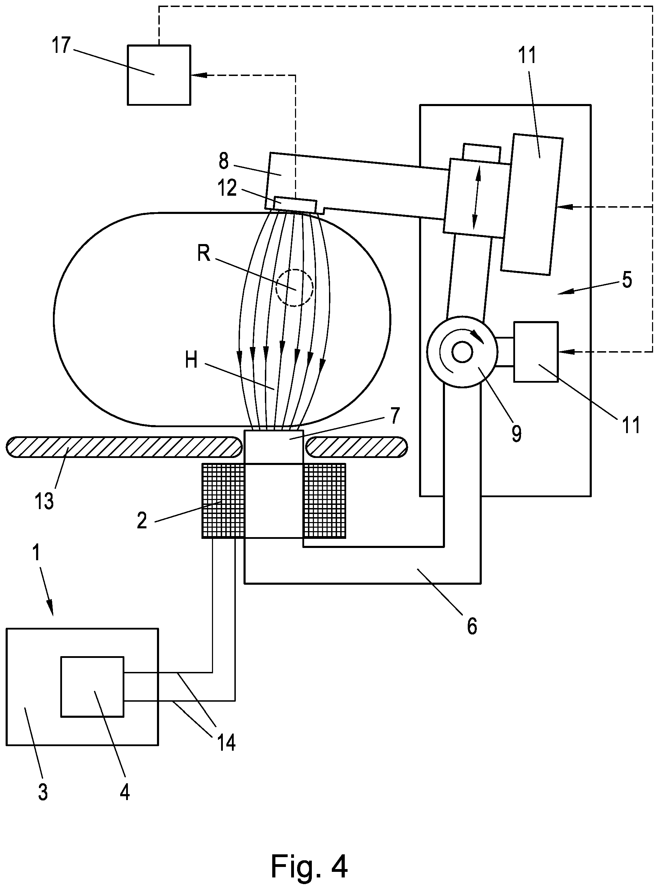

Fig. 2 zeigt ein Blockschaltbild einer Ausführungsvariante der erfindungsgemäßen magnetischen Stimulationsvorrichtung 1 mit zwei als Luftspulen ausgeführten Magnetspulen 2, 2'. Die beiden Magnetspulen 2, 2' sind über die Verstellvorrichtung 5 miteinander verbunden und so angeordnet, dass die zu stimulierende Körperregion R zwischen den Magnetspulen 2, 2' anordenbar ist. Durch die Verstellvorrichtung 5 kann eine optimale Anpassung der Lage der Magnetspulen 2, 2' in Bezug auf die zu stimulierende Körperregion R erzielt werden. Im Falle der Konstruktion mit zwei gegenüberliegenden Magnetspulen 2, 2' ist die Verstellvorrichtung 5 vorzugsweise aus magnetisch nicht leitfähigem bzw. magnetisch isolierendem Material, beispielsweise Kunststoff, gebildet, um keinen magnetischen Kurzschluss der von den Magnetspulen 2, 2' ausgehenden Feldlinien zu verursachen. Die Verstellvorrichtung 5 kann ein Drehgelenk 9 und bzw. oder ein Schiebegelenk 10 beinhalten. Durch die Verstellmöglichkeit zumindest einer Magnetspule 2, 2' kann das Magnetfeld H auch besser in der zu stimulierenden Region R konzentriert werden, da die Magnetspulen 2, 2' näher an diese Körperregion R herangeführt werden können. Beispielsweise kann die obere Magnetspule 2 im Falle der Stimulation des Oberkörpers auch etwas in das Haut- und Fettgewebe der jeweiligen Körperregion R eingedrückt werden. Bei der Stimulation von sich bewegenden Körperteilen, beispielsweise dem Thorax, ist auch eine aktive (sensorgesteuerte) oder passive Nachführung der zumindest einen Magnetspule 2, 2' an die bewegte Körperregion, beispielsweise synchron mit der Atmung, denkbar. Dabei ist eine automatisierte und angetriebene Verstellvorrichtung 5 vorgesehen, bei der das Drehgelenk 9 und bzw. oder Schiebegelenk 10 mit einem Antrieb 11 verbunden ist und synchron zur Atmung die Bewegung gesteuert wird (s. Fig. 4).

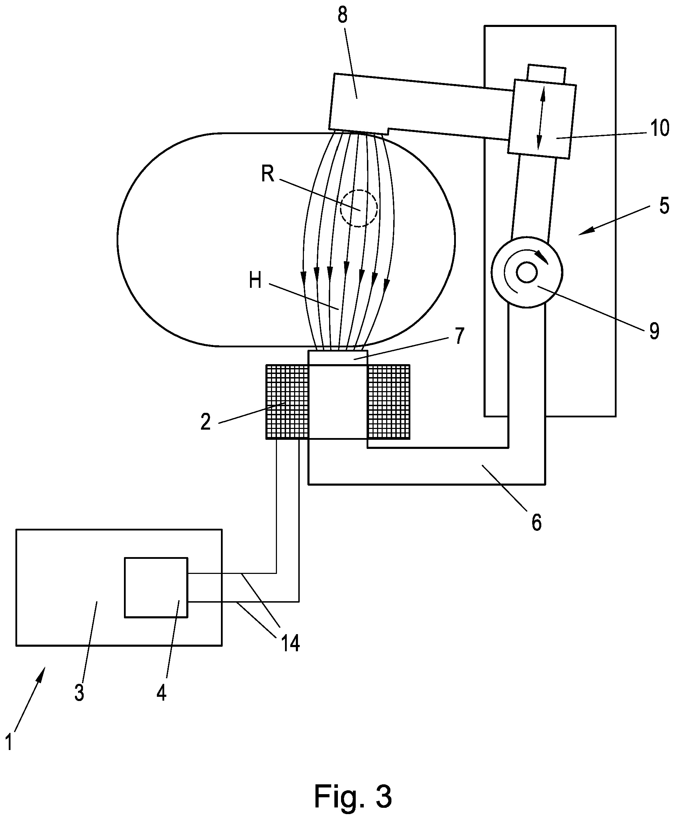

In Fig. 3 ist ein Blockschaltbild einer weiteren Ausführungsvariante der erfindungsgemäßen magnetischen Stimulationsvorrichtung 1 mit einer Magnetspule 2 und einem Magnetjoch 6 dargestellt. In diesem Fall sind die Pole 7, 8 des Magnetjochs 6 über die Verstellvorrichtung 5 verstellbar miteinander verbunden und schließen die zu stimulierende Körperregion R ein. Zur Sicherstellung eines geschlossenen Magnetkreises sind in diesem Fall die Komponenten der Verstellvorrichtung 5 aus magnetisch leitfähigem Material, beispielsweise Eisen, gebildet. Auch im Falle dieser Ausführungsvariante besteht die Verstellvorrichtung 5 aus einem Drehgelenk 9 und bzw. oder einem Schiebegelenk 10..

In Fig. 4 ist eine Prinzipskizze einer Liege 13 mit einer erfindungsgemäßen magnetischen Stimulationsvorrichtung 1 wiedergegeben. Die magnetische Stimulationsvorrichtung 1 ist aus zumindest einer Magnetspule 2 und einem Magnetjoch 6 gebildet und so gestaltet, dass die Pole 7, 8 des Magnetjochs 6 im Wesentlichen gegenüberliegend angeordnet sind, sodass die zu stimulierende Körperregion R zwischen den Polen 7, 8 des Magnetjochs 6 angeordnet werden können. Die Magnetspule 2 bzw. ein Magnetpol 7 ist unterhalb der Liege 13 bzw. in einer darin befindlichen Aussparung angeordnet. Der Patient bzw. die zu stimulierende Körperregion R liegt auf der Liege 13 auf, wodurch eine Stimulation auch im liegenden Zustand bzw. im Tiefschlaf bei Intensivpatienten ermöglicht wird. Die Verstellvorrichtung 5 ist ähnlich der Variante gemäß Fig. 3 aufgebaut, wobei das Drehgelenk 9 und das Schiebegelenk 10 mit jeweils einem Antrieb 11 verbunden ist, um eine automatische Verstellung zu ermöglichen. Zu diesem Zweck sind die Antriebe 11 mit einer entsprechenden Antriebssteuerung 17 verbunden. Um eine unzulässig hohe Druckausübung auf die zu stimulierende Körperregion R zu vermeiden, sind Sensoren 12 beispielsweise an den Polen 7, 8 des Magnetjochs 6 angeordnet, die mit der Antriebssteuerung 17 verbunden sein können. Als Sensoren 12 kommen verschiedene Drucksensoren, optische Sensoren oder auch Atemflowsensoren zu Erfassung der Spontanatmung des Patienten zur Anwendung.

Schließlich zeigt Fig. 5 eine Detailansicht einer magnetischen Stimulationsvorrichtung 1 mit Elementen zur Konzentration des Magnetfelds H, die beispielsweise durch geeignet angeordnete Permanentmagnete 16 gebildet sein können.

## Patentansprüche

1. Vorrichtung (1) zur magnetischen Stimulation von Regionen (R) eines menschlichen oder tierischen Körpers, mit zumindest zwei als Luftspulen ausgebildeten Magnetspulen (2, 2') oder zumindest einer auf einem Magnetjoch (6) angeordneten Magnetspule (2, 2'), welche mit einem Stimulator (3) verbunden sind, der einen Leistungsteil (4) zur Erzeugung an die zumindest eine Magnetspule (2, 2') anzulegende elektrische Impulse (I) aufweist, wobei die zumindest zwei als Luftspulen ausgebildeten Magnetspulen (2, 2') oder die Pole (7, 8) des Magnetjochs (6) beiderseits der zu stimulierenden Körperregion (R) anordenbar sind, sodass das in der zumindest einen Magnetspule (2, 2') erzeugte Magnetfeld (H) in der Körperregion (R) induzierbar ist, wobei die zumindest zwei als Luftspulen ausgebildeten Magnetspulen (2, 2') oder die Pole (7, 8) des Magnetjochs (6) über eine Verstellvorrichtung (5) gegenüber der zu stimulierenden Körperregion (R) verstellbar und derart ausgebildet sind, sodass die zu stimulierende Körperregion (R) innerhalb des induzierten Magnetfelds (H) anordenbar ist, wobei die Verstellvorrichtung (5) ein Drehgelenk (9) und bzw. oder ein Schiebegelenk (10) beinhaltet, **dadurch gekennzeichnet, dass** die zumindest zwei als Luftspulen ausgebildeten Magnetspulen (2, 2') oder die Pole (7, 8) des Magnetjochs (6) über die Verstellvorrichtung (5) verstellbar miteinander verbunden sind, sodass eine Anpassung an die Größe der zu stimulierenden Körperregion (R) erzielbar ist, und dass zumindest ein Antrieb (11) zur Verstellung des Drehgelenks (9) und bzw. oder des Schiebegelenks (10) vorgesehen ist, welcher zumindest eine Antrieb (11) mit zumindest einem Sensor (12) zur Erfassung eines Drucks auf die zu stimulierende Körperregion (R) und einer Bewegung der zu stimulierenden Körperregion (R) verbunden ist, für eine automatische, aktive Nachführung der zumindest einen Magnetspule (2, 2') oder der Pole (7, 8) des Magnetjochs (6) synchron an die zu stimulierende, bewegte Körperregion (R).

2. Magnetische Stimulationsvorrichtung (1) nach Anspruch 1, **dadurch gekennzeichnet, dass** die Verstellvorrichtung (5) aus magnetisch nicht leitfähigem (magnetisch isolierendem) Material, insbesondere Kunststoff, gebildet ist.

3. Magnetische Stimulationsvorrichtung (1) nach Anspruch 1, **dadurch gekennzeichnet, dass** die Verstellvorrichtung (5) aus magnetisch leitfähigem Material, beispielsweise Eisen, gebildet ist.

4. Magnetische Stimulationsvorrichtung (1) nach einem der Ansprüche 1 bis 3, **dadurch gekennzeichnet, dass** zumindest ein Sensor (12) durch einen optischen Sensor gebildet ist.

5. Magnetische Stimulationsvorrichtung (1) nach einem der Ansprüche 1 bis 4, **dadurch gekennzeichnet, dass** zumindest ein Sensor (12) durch einen Atemflowsensor gebildet ist.

6. Magnetische Stimulationsvorrichtung (1) nach einem der Ansprüche 1 bis 5, **dadurch gekennzeichnet, dass** eine Liege (13) oder ein Sitz (14) vorgesehen ist, wobei unterhalb der Liege (13) oder des Sitzes (14) zumindest eine Magnetspule (2) angeordnet ist.

7. Magnetische Stimulationsvorrichtung (1) nach einem der Ansprüche 1 bis 6, **dadurch gekennzeichnet, dass** Elemente (15) zum Konzentrieren des Magnetfelds (H), beispielsweise Permanentmagnete (16), vorgesehen sind.

## Claims

1. Device (1) for magnetically stimulating areas (R) of a human body or animal body, having at least two magnetic coils (2, 2') which are embodied as air coils, or at least one magnetic coil (2, 2') which is arranged on a magnet yoke (6) and is connected to a stimulator (3) which has a power component (4) for generating electrical pulses (I) which are to be applied to the at least one magnetic coil (2, 2'), wherein the at least two magnetic coils (2, 2') which are embodied as air coils, or the poles (7, 8) of the magnet yoke (6), can be arranged on either side of the body area (R) which is to be stimulated, with the result that the magnetic field (H) which is generated in the at least one magnetic coil (2, 2') can be induced in the body area (R), wherein the at least two magnetic coils (2, 2') which are embodied as air coils, or the poles (7, 8) of the magnet yoke (6), can be adjusted, by means of an adjustment device (5), with respect to the body area (R) which is to be stimulated and are embodied in such a way that the body area (R) which is to be stimulated can be arranged within the induced magnetic field (H), wherein the adjustment device (5) contains a rotary joint (9) and/or a sliding joint (10), **characterized in that** the at least two magnetic coils (2, 2') which are embodied as air coils, or the poles (7, 8) of the magnet yoke (6), are connected to one another so as to be adjustable by means of the adjustment device (5), with the result that adaptation can be carried out to the size of the body area (R) which is to be simulated, and **in that** at least one drive (11) is provided for adjusting the rotary joint (9) and/or the sliding joint (10), which at least one drive (11) is connected to at least one sensor (12) for sensing the pressure on the body area (R) which is to be stimulated, and a movement of the body area (R) which is to be stimulated, for automatic active tracking of the at least one magnetic coil (2, 2') or of the poles (7, 8) of the magnet yoke (6) synchronously to the moved body area (R) which is to be stimulated.

2. Magnetic stimulation device (1) according to Claim 1, **characterized in that** the adjustment device (5) is formed from magnetically non-conducting (magnetically insulated) material, in particular plastic.

3. Magnetic stimulation device (1) according to Claim 1, **characterized in that** the adjustment device (5) is formed from magnetically conducting material, for example iron.

4. Magnetic stimulation device (1) according to one of Claims 1 to 3, **characterized in that** at least one sensor (12) is formed by an optical sensor.

5. Magnetic stimulation device (1) according to one of Claims 1 to 4, **characterized in that** at least one sensor (12) is formed by a breath flow sensor.

6. Magnetic stimulation device (1) according to one of Claims 1 to 5, **characterized in that** a bunk (13) or a seat (14) is provided, wherein at least one magnetic coil (2) is arranged underneath the bunk (13) or the seat (14).

7. Magnetic stimulation device (1) according to one of Claims 1 to 6, **characterized in that** elements (15) for concentrating the magnetic field (H), for example permanent magnets (16), are provided.

## Revendications

1. Dispositif (1) pour la stimulation magnétique de régions (R) d'un corps humain ou animal, avec au moins deux bobines magnétiques (2, 2'), conçues comme des bobines à air, ou au moins une bobine magnétique (2, 2') disposée sur une culasse magnétique (6), qui sont reliées avec un simulateur (3), qui comprend une partie de puissance (4) pour la génération d'impulsions électriques (I) à appliquer à l'au moins une bobine magnétique (2, 2'), les au moins deux bobines magnétiques (2, 2') conçues comme des bobines à air ou les pôles (7, 8) de la culasse magnétique (6) pouvant être disposés des deux côtés de la région du corps (R) à stimuler, de façon à ce que le champ magnétique (H) généré dans l'au moins une bobine magnétique (2, 2') puisse être induit dans la région du corps (R), les au moins deux bobines magnétiques (2, 2') conçues comme des bobines à air ou les pôles (7, 8) de la culasse magnétique (6) pouvant être déplacés par rapport à la région du corps (R) à stimuler par l'intermédiaire d'un dispositif de déplacement (5) et étant conçus de façon à ce que la région du corps (R) à stimuler puisse être disposée à l'intérieur du champ magnétique induit (H), le dispositif de déplacement (5) contenant une articulation rotative (9) et/ou une articulation coulissante (10), **caractérisé en ce que** les au moins deux bobines magnétiques (2, 2') conçues comme des bobines à air ou les pôles (7, 8) de la culasse magnétique (6) sont reliés entre eux de manière mobile par l'intermédiaire du dispositif de déplacement (5), de façon à permettre une adaptation à la taille de la région du corps (R) à stimuler, et **en ce qu'**au moins un dispositif d'entraînement (11) pour le déplacement de l'articulation rotative (9) et/ou de l'articulation coulissante (10) soit prévu, cet au moins un dispositif d'entraînement (11) étant relié avec au moins un capteur (12) pour la mesure d'une pression sur la région du corps (R) à stimuler et d'un déplacement de la région du corps (R) à stimuler, pour un suivi automatique actif de l'au moins une bobine magnétique (2, 2') ou des pôles (7, 8) de la culasse magnétique (6) de manière synchrone au niveau de la région du corps (R) à stimuler déplacée.

2. Dispositif de stimulation magnétique (1) selon la revendication 1, **caractérisé en ce que** le dispositif de déplacement (5) est constitué d'un matériau magnétiquement non conducteur (magnétiquement isolant), plus particulièrement d'une matière plastique.

3. Dispositif de stimulation magnétique (1) selon la revendication 1, **caractérisé en ce que** le dispositif de déplacement (5) est constitué d'un matériau magnétiquement conducteur, par exemple de fer.

4. Dispositif de stimulation magnétique (1) selon l'une des revendications 1 à 3, **caractérisé en ce qu'**au moins un capteur (12) est constitué d'un capteur optique.

5. Dispositif de stimulation magnétique (1) selon l'une des revendications 1 à 4, **caractérisé en ce qu'**au moins un capteur (12) est constitué d'un capteur de débit respiratoire.

6. Dispositif de stimulation magnétique (1) selon l'une des revendications 1 à 5, **caractérisé en ce qu'**une chaise longue (13) ou un siège (14) est prévu, au moins une bobine magnétique (2) étant disposée en dessous de la chaise longue (13) ou du siège (14).

7. Dispositif de stimulation magnétique (1) selon l'une des revendications 1 à 6, **caractérisé en ce que** des éléments (15) pour la concentration du champ magnétique (H), par exemple des aimants permanents (16), sont prévus.
